(19) Europäisches Patentamt
European Patent Office
Office européen des brevets

(11) Veröffentlichungsnummer : **0 111 124**
**B1**

(12) # EUROPÄISCHE PATENTSCHRIFT

(45) Veröffentlichungstag der Patentschrift : **16.04.86**

(21) Anmeldenummer : **83110606.7**

(22) Anmeldetag : **24.10.83**

(51) Int. Cl.[4] : **C 07 C 59/64**, C 07 C 69/734, C 07 C103/58, C 07 C154/00, C 07 C 43/225, C 07 F 9/52, C 07 C 47/277

(54) **Polyenverbindungen, ihre Herstellung und diese Verbindungen enthaltende pharmazeutische Präparate.**

(30) Priorität : **05.11.82 US 439516**

(43) Veröffentlichungstag der Anmeldung : **20.06.84 Patentblatt 84/25**

(45) Bekanntmachung des Hinweises auf die Patenterteilung : **16.04.86 Patentblatt 86/16**

(84) Benannte Vertragsstaaten : **AT BE CH DE FR GB IT LI LU NL SE**

(56) Entgegenhaltungen :
**US-A- 4 054 589**
**US-A- 4 266 073**

(73) Patentinhaber : **F. HOFFMANN-LA ROCHE & CO. Aktiengesellschaft**

**CH-4002 Basel (CH)**

(72) Erfinder : **Pawson, Beverley Ann**
**7 Belleclaire Place**
**Verona, N.J. (US)**

(74) Vertreter : **Lederer, Franz, Dr. et al**
**Patentanwälte Dr. Lederer Franz Meyer-Roxlau Reiner F. Lucile-Grahn-Strasse 22**
**D-8000 München 80 (DE)**

**EP 0 111 124 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Polyenverbindungen, ein Verfahren zu deren Herstellung und diese Verbindung enthaltende pharmazeutische Präparate.

Die erfindungsgemässen Polyenverbindungen haben die allgemeine Formel

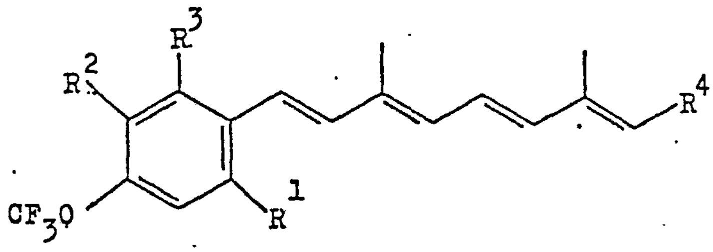 (I)

worin

$R^1$ nieder-Alkyl oder Halogen,

$R^2$ nieder-Alkyl,

$R^3$ nieder-Alkyl oder Halogen,

$R^4$ —$CH_2$—OH, 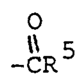 oder 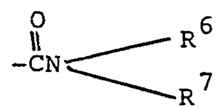

$R^5$ Wasserstoff, Hydroxy oder nieder-Alkoxy

und $R^6$ und $R^7$ Wasserstoff oder nieder-Alkyl bedeuten.

Die Erfindung betrifft weiterhin pharmazeutisch anwendbare Salze von Carbonsäuren der Formel I.

Der hier verwendete Ausdruck nieder-Alkyl bezieht sich auf geradkettige oder verzweigte Alkylgruppen mit 1 bis 7 C-Atomen, z. B. Methyl, Aethyl, n-Propyl und Isopropyl. Nieder-Alkoxy bezeichnet geradkettige oder verzweigte Alkoxygruppen mit 1 bis 7 C-Atomen, z. B. Methoxy, Aethoxy und Isopropoxy. Aryl bezeichnet ein- oder mehrkernige aromatische Kohlenwasserstoffreste, die unsubstituiert oder in einer oder mehreren Stellungen durch Halogen, Nitro, nieder-Alkyl oder nieder-Alkoxy substituiert sein können. Typische Arylgruppen sind Phenyl, Benzyl, Naphthyl, Anthranyl, Phenanthranyl und Azulyl die unsubstituiert oder durch eine oder mehrere der vorgenannten Substituenten substituiert sein können.

Halogen bezeichnet Chlor, Brom oder Jod. Alkalimetalle sind Lithium, Natrium, Kalium und rubidium. Erdalkalimetalle sind Beryllium, Magnesium, Calcium und Strontium. Beispiele für pharmazeutisch anwendbare Salze sind Alkalimetallsalze, Erdalkalimetallsalze und substituierte oder unsubstituierte Ammoniumsalze. Als Anion einer organischen Säure wird das Anion einer organischen Verbindung bezeichnet, die eine saure Gruppe trägt wie eine Carboxylgruppe, eine Sulfonsäuregruppe, eine Hydroxamsäuregruppe oder Sulfamsäuregruppe. Ein bevorzugtes Anion ist das Tosyloxyion.

Sofern nicht anders angegeben, umfassen alle Formeln cis/trans-Gemische und die entsprechenden cis- und trans- Verbindungen.

Die Alltransverbindungen der Formel I sind bevorzugt.

Die Verbindungen der Formel I und ihre Salze können erfindungsgemäss dadurch hergestellt werden, dass man eine Verbindung der Formel

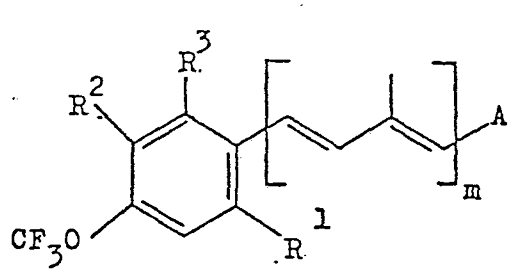 (II)

mit einer Verbindung der Formel

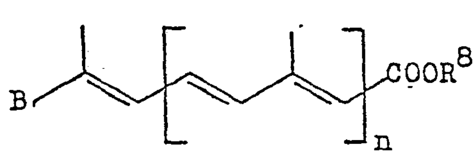 (III)

worin $R^1$, $R^2$ und $R^3$ die obige Bedeutung haben, eines der Symbole A und B Formyl ist und das andere eine Gruppe der Formeln

$$-CH_2-\overset{\oplus}{P}(Ar)_3Y^{\ominus} \quad \text{oder} \quad -CH_2-\overset{O}{\overset{\|}{P}}(OR^9)_2$$

darstellt, Ar Aryl ist, $Y^{\ominus}$ das Anion einer organischen oder anorganischen Säure und $R^8$ und $R^9$ nieder-Alkyl sind, wobei m = 0 und n = 1 oder m = 1 und n = 0 sind, umsetzt und gewünschtenfalls eine nieder-Alkoxycarbonylgruppe $-COOR^8$ in einer erhaltenen Verbindung der Formel I in eine Carboxyl, Formyl, Hydroxymethyl, Carboxamid oder mono- oder Dialkylcarboxamidgruppe umwandelt und gewünschtenfalls eine erhaltene Carbonsäure in ein pharmazeutisch anwendbares Salz umwandelt.

Erfindungsgemäss werden die Verbindungen der Formel I durch Umsetzung eines Aldehyds der Formel II mit einem Phosphoniumsalz oder einem Phosphonat der Formel III oder durch Umsetzung eines Phosphoniumsalzes oder Phosphonats der Formel II mit einem Aldehyd der Formel III erhalten. Diese Reaktionen können unter den Bedingungen der Wittig- und bzw. Horner-Reaktion durchgeführt werden.

Phosphoniumsalze der Formel II oder III können mit dem entsprechenden Aldehyd in einer Wittig-Reaktion zu einer Verbindung der Formel I umgesetzt werden in der $R^4$

$$-\overset{O}{\overset{\|}{C}}R^5$$

und $R^5$ nieder-Alkoxy ist.

In der Wittig-Reaktion werden die Verbindung in Gegenwart eines anorganischen oder organischen säurebindenden Mittels umgesetzt. Beispiele solcher Mittel sind Alkyllithium z. B. n-Butyllithium und Methyllithium, Alkalimetallhydroxide und Alkoholate, z. B. Natriumhydroxid und Natriummethylat, tert. Amine, z. B. Triäthylamin und Pyridin und alkalische Oxide, die alkylsubstituiert sein können, z. B. Aethylenoxid und 1,2-Butylenoxid. Obschon nicht notwendig, kann die Reaktion in einem inerten Lösungsmittel wie Diäthyläther und Tetrahydrofuran oder einem aliphatischen oder aromatischen Kohlenwasserstoff wie Hexan, Benzol oder Toluol durchgeführt werden. Die Reaktionstemperatur ist nicht kritisch, im allgemeinen verläuft die Reaktion zwischen etwa —50 und etwa + 60 °C. Eine Reaktionstemperatur zwischen etwa —45 °C und etwa —25 °C ist bevorzugt.

In einer anderen Ausführungsform des erfindungsgemässen Verfahrens wird ein Phosphonat der Formel II oder III mit dem entsprechenden Aldehyd in einer Horner-Reaktion umgesetzt. Dieses Verfahren verläuft im allgemeinen under Anwendung einer Base, wie einem Alkalimetall oder Erdalkalimetallhydroxid, z. B. Natriumhydroxid oder einem Alkalimetallakoholat, z. B. Natriummethylat. Vorzugsweise wird das Verfahren in Gegenwart eines inerten organischen Lösungsmittels wie Benzol, Toluol, Dimethylformamid oder Tetrahydrofuran durchgeführt. Obschon die Temperatur nicht kritisch ist, wird die Horner-Reaktion im allgemeinen bei Temperaturen zwischen etwa 20 °C und etwa 110 °C vorzugsweise bei etwa 60 °C durchgeführt.

Zur Herstellung von Verbindungen der Formel I, worin

$$R^4 \;\; -CH_2OH, \;\; -\overset{O}{\overset{\|}{C}}R^5 \;\; \text{oder} \;\; -\overset{O}{\overset{\|}{C}}N\begin{matrix} R^6 \\ R^7 \end{matrix},$$

$R^5$ Wasserstoff oder Hydroxy und $R^6$ und $R^7$ Wasserstoff oder nieder-Alkyl darstellen, können die üblichen Verfahren angewandt werden. Beispielsweise kann ein Carbonsäureester der Formel I zum entsprechenden Alkohol der Formel I ($R^4$ = $-CH_2OH$) reduziert werden. Der Alkohol kann zu dem entsprechenden Aldehyd der Formel I ($R^4$ =

$$-\overset{O}{\overset{\|}{C}}R^5,$$

$R^5$ = Wasserstoff) oxydiert werden. Ein Carbonsäureester der Formel I kann zur entsprechenden Carbonsäure der Formel I hydrolysiert werden und die Carbonsäure der Formel I kan in ein unsubstituiertes oder nieder-Alkyl substituiertes Amid der Formel I

$$(R^4 = -\overset{O}{\overset{\|}{C}}N\begin{matrix} R^6 \\ R^7 \end{matrix},$$

$R^6$ und $R^7$ Wasserstoff oder nieder-Alkyl) umgewandelt werden.

Zur Umwandlung eines Carbonsäureesters in einen Alkohol können die üblichen Verfahren zur

Reduktion eines Esters zu einem Alkohol angewandt werden. Vorzugsweise setzt man den Ester mit Diisobutylaluminiumhydrid in einem ätherischen Lösungsmittel um. Obschon die Temperatur nicht kritisch ist, arbeitet man im allgemeinen bei Temperaturen zwischen etwa —70 °C und etwa + 40 °C, wobei eine Temperatur von etwa 0 °C bevorzugt ist.

Ein Alkohol der Formel I kann zum Aldehyd mittels an sich bekannter Techniken für die Oxydation eines Alkohols zum Aldehyd oxydiert werden. Ein geeignetes Oxydationsmittel ist Mangandioxid.

Ein Carbonsäureester kann durch saure oder basische Hydrolyse in eine Carbonsäure umgewandelt werden. Hierfür kommen die konventionellen Hydrolysemethoden in Betracht. Beispielsweise kann man einen Ester der Formel I mit einem Alkalimetall- oder Erdalkalimetallhydroxid, z. B. Kaliumhydroxid in Gegenwart eines wässrigen Alkohols, z. B. Methanol oder Propanol umsetzen. Die Reaktionstemperatur ist nicht kritisch und kann zwischen etwa 50 °C und etwa 100 °C variieren, wobei eine Temperatur von etwa 80 °C bevorzugt ist.

Eine Carbonsäure der Formel I kann in ein unsubstituiertes oder nieder-Alkyl-substituiertes Amid der Formel I nach konventionellen Methoden zur Amidierung einer Carbonsäure umgewandelt werden. Vorzugsweise wird die Säure mit einem geeigneten unsubstituierten oder nieder-Alkyl substituierten Amin in Gegenwart von Carbonyldiimidazol umgesetzt, wobei man das entsprechende unsubstituierte oder nieder-Alkyl substituierte Amid erhält. Vorzugsweise führt man die Reaktion in einem polaren organischen Lösungsmittel wie Tetrahydrofuran oder Diäthyläther aus.

Die Verbindungen der Formel I können als cis/trans- Gemisch auftreten, das in an sich bekannter Weise, z. B. durch Chromatographie in die entsprechenden cis- und trans-Enantiomeren aufgetrennt werden kann oder in an sich bekannter Weise zu den all-trans-Verbindungen isomerisiert werden kann.

Alle konventionellen Methoden zu Isomerisierung von Doppelbindungen können angewandt werden um die all-trans-Verbindung zu bilden. Beispielsweise kann ein 8-cis/trans-Gemisch einer Verbindung der Formel I mit katalytischen Mengen Jod in einem organischen Lösungsmittel, z. B. Benzol oder Toluol behandelt werden, wobei man das gewünschte all-trans-Produkt erhält. Obschon die Temperatur nicht kritisch ist, verläuft die Isomerisierung im allgemeinen zwischen etwa 10 °C bis etwa 60 °C, wobei Temperaturwerte von etwa 25 °C bis etwa 35 °C bevorzugt sind.

Die Reaktionspartner der Formel II und III sind bekannt oder können aus bekannten Verbindungen mit konventionellen Verfahren wie in Schema 1 angegeben hergestellt werden.

Schema 1

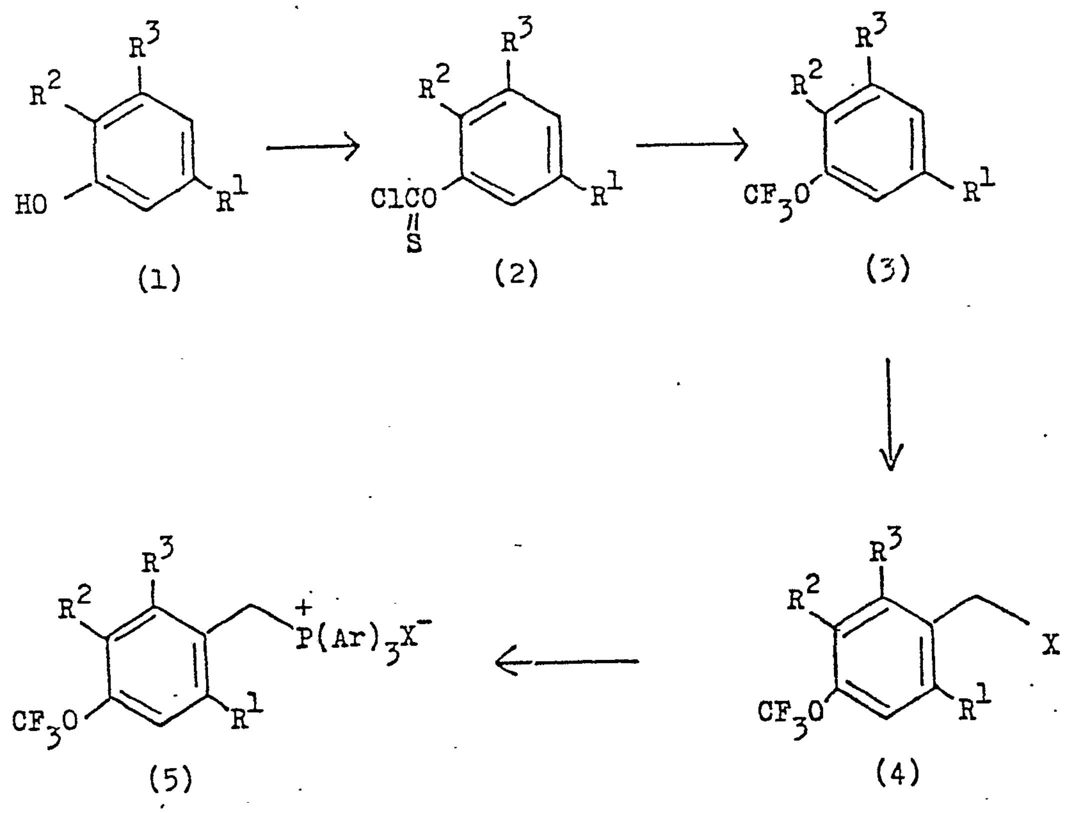

Im obigen Reaktionsschema haben $R^1$, $R^2$ und $R^3$ die früher angegebene Bedeutung, X ist Halogen und Ar ist Aryl.

Gemäss Schema 1 wird eine Verbindung der Formel I in eine Verbindung der Formel II durch konventionelle Derivatisierung eines Phenols zum Chlorthionocarbonats umgewandelt. Eine geeignete

Methode besteht darin, dass man die Verbindung I in einer Base wie Natriumhydroxid und einem nicht Wässrigen polaren organischen Lösungsmittel wie Tetrachlorkohlenstoff oder Chloroform umsetzt. Die Temperatur ist nicht kritisch, die Reaktion verläuft im allgemeinen zwischen etwa —20 °C und etwa + 20 °C, vorzugsweise bei etwa —10 °C.

Die Verbindung II wird dann in eine Verbindung III durch Umsetzung mit Molybdänhexafluorid, vorzugsweise in einem polaren organischen Lösungsmittel nach Mathey-Bensoam umgewandelt. Als Lösungsmittel kommen insbesondere chlorierte Lösungsmittel wie Methylenchlorid in Betracht. Die Temperatur ist nicht kritisch, die Reaktion verläuft im allgemeinen zwischen etwa —50 °C und etwa 0 °C, vorzugsweise bei etwa —25 °C.

Die Verbindung III wird in die Verbindung IV mittels konventioneller Halogenmethylierungsverfahren umgewandelt. Beispielsweise wird eine Verbindung III mit einem Halogenmethylmethyläther, insbesondere Chlormethylmethyläther, in einem Gemisch von Essigsäure, Salzsäure, und wässrigem Formaldehyd zu einer Verbindung IV umgesetzt. Die Temperatur ist nicht kritisch, die Reaktion verläuft im allgemeinen zwischen etwa 50 °C und etwa 100 °C, vorzugsweise bei etwa 85 °C.

Die Verbindung IV wird zu einer Verbindung V in für die Herstellung von substituierten Phosphoniumsalzen an sich bekannter Weise umgewandelt. Beispielsweise wird die Verbindung IV mit einem Triarylphosphin umgesetzt. Beispiele von Triarylphosphinen sind Triphenylphosphin und Tritolylphosphin. Triphenylphosphin ist bevorzugt. Die Reaktion wird vorzugsweise in einem Lösungsmittel wie einem polaren organischen Lösungsmittel, z. B. Toluol, Aethylacetat und insbesondere Acetonitril ausgeführt. Die Temperatur ist nicht kritisch und kann zwischen etwa 50 °C bis etwa 100 °C liegen, vorzugsweise beträgt die Temperatur etwa 80 °C. Gewünschtenfalls kann das Halogenidanion in dem so erhaltenen Phosphoniumhalogenid gegen ein organisches Anion ausgetauscht werden, beispielsweise durch Behandlung mit Silbertosylat wobei man ein Silberhalogenid und eine Verbindung II erhält, worin Y das Tosyloxyion ist.

Die Verbindung (4) kann in ein Phosphonat der Formel II worin m = 0 ist in einer konventionellen Michaelis-Arbuzow-Reaktion mit einem tri(nieder) Alkylphosphit, z. B. Triäthylphosphit umgewandelt werden. Obschon die Temperatur nicht kritisch ist verläuft die Reaktion im allgemeinen zwischen etwa 35 °C und 150 °C wobei etwa 100 °C bevorzugt sind.

Die Verbindung (3) kann formyliert werden wobei man eine Verbindung der Formel II, worin m = 0 ist und A Formyl ist erhält. Dies kann durch Umsetzung der Verbindung (3) mit einem Formylierungsmittel in Gegenwart einer Lewis-Saüre erreicht werden. Als Formylierungsmittel können insbesondere Orthoameisensäureester, Formylchlorid und Dimethylformamid verwendet werden. Besonders geeignete Lewis-Säuren sind die Halogenide von Zink, Aluminium, Titan, Zinn und Eisen wie Zinkchlorid, Aluminiumtrichlorid, Titantetrachlorid, Zinntetrachlorid und Eisentrichlorid wie auch die Halogenide organischer und anorganischer Säuren wie beispielsweise Phosphoroxychlorid und Methansulfochlorid.

Wenn das Formylierungsmittel im Ueberschuss anwesend ist, kann die Formylierung ohne Zusatz eines weiteren Lösungsmittels durchgeführt werden. Im allgemeinen ist es jedoch empfehlenswert die Formylierung in einem inerten Lösungsmittel, z. B. Nitrobenzol oder einem chlorierten Kohlenwasserstoff wie Methylenchlorid vorzunehmen. Die Formylierung kann bei Temperaturen zwischen 0 °C und dem Siedepunkt des Gemisches ausgeführt werden.

Der erhaltene substituierte Benzaldehyd kann anschliessend in an sich bekannter Weise einer Kettenverlängerung unterworfen werden, wobei man mit Aceton in der Kälte (d. h. bei Temperaturen von etwa 0 °C bis 30 °C) in Gegenwart von Alkali (z. B. verdünnter Natronlauge) zu einem substituierten Phenyl-but-3-en-2-on umsetzt, das in das entsprechende substituierte Phenyl-3-methyl-3-hydroxy-penta-4-en-1-yn in an sich bekannter Weise mittels einer metallorganischen Reaktion (z. B. mit einer Acetylen-Grignardreaktion) umgewandelt wird. Das erhaltene tert. acetylenische Carbinol wird anschliessend partiell hydriert, wobei man einen partiell deaktivierten Edelmetallkatalysator (Lindlar-Katalysator) verwendet. Das erhaltene tert. Aethylencarbinol kann anschliessend unter Allylumlagerung in ein Phosphoniumsalz der Formel II, worin m = 1 ist umgewandelt werden, durch Behandlung mit einem Triarylphosphin, insbesondere Triphenylphosphin in Gegenwart eines Wasserstoffhalogenids wie Chlorwasserstoff oder Bromwasserstoff in einem Lösungysmittel, z. B. Benzol. Das tert. Aethylencarbinol kann weiterhin zu einem Halogenid halogeniert werden, das mit einem Trialkylphosphit, z. B. Triäthylphosphit das entsprechende Phosphonat der Formel II, worin m = 1 ist, liefert.

Verbindungen der Formel II, worin m = 1 ist und A eine Formylgruppe darstellt, können beispielsweise dadurch hergestellt werden, dass man ein substituiertes Phenyl-but-3-en-2-on einer Wittig-Reaktion mit Aethoxycarbonylmethylentriphenylphosphoran oder mit Diäthylphosphonoessigsäureäthylester unterwirft. Der erhaltene substituierte Phenyl-3-methyl-penta-2,4-dien-1-säureäthylester wird anschliessend in der Kälte mit einem komplexen Metallhydrid, insbesondere Lithiumaluminiumhydrid, in einem organischen Lösungsmittel, z. B. Aether oder Tetrahydrofuran zu einem substituierten Phenyl-3-methyl-penta-2,4-dien-1-ol reduziert. Dieser Alkohol wird dann durch Behandlung mit einem Oxidationsmittel, beispielsweise Mangandioxid in einem organischen Lösungsmittel, wie Aceton oder Methylenchlorid, bei einer Temperatur zwischen 0 °C und dem Siedepunkt des Gemisches, oxidiert, wobei man das gewünschte substituierte Phenyl-3-methyl-penta-2,4-dien-1-al der Formel II (m = 1, A = CHO) erhält.

Die Verbindung der Formel I und deren Salze sind pharmakodynamisch wirksam. Sie bewirken die

Regression des Wachstums von Tumoren wie Papillomen und die Regression des Wachstums von Chondrosarcomen.

Die Verbindungen der Formel I können auch als Medikamenter zur topischen und systemischen Therapie von Akne, Psoriasis und anderen verwandten dermatologischen Erkrankungen verwendet werden die durch eine vermehrte oder pathologisch veränderte Verhornung gekennzeichnet sind, wie auch bei entzündlichen und allergischen dermatologischen Zuständen. Sie können weiterhin verwendet werden, um entzündliche oder degenerative Veränderungen der Schleimhäute zu behandeln.

Zur Prüfung ihrer pharmakodynamischen Eigenschaften wurden die erfindungsgemässen Verbindungen dem Hautpapillomtest (Experientia Vol. 27 (1971) Seiten 90 ff) und dem Vitamin A Hypervitaminosetest (Europ. J. Cancer, Vol. 10 (1974) Seiten 731-737) unterworfen. Die Resultate sind in Tabelle 1 angegeben.

Der therapeutische Index ist definiert als Quotient der niedrigsten täglichen Dosis, die eine Vitamin-A Hypervitaminose hervorruft (Zähler) und der niedrigsten Dosis die etwa 50 % Regression des Papillomdurchmessers hervorruft (Nenner). Je höher der therapeutische Index für eine bestimmte Verbindung ist, desto günstiger ist das Verhältnis zwischen der antineoplastischen Aktivität (Regression der Papillome) und der Toxizität (Vitamin-A-Hypervitaminose) der betreffenden Verbindung. Ein derart günstiges Verhältnis ist ein Anzeichen, dass die Verbindung als Antitumormittel wirksam ist.

(Siehe Tabelle 1 Seite 7 f.)

Tabelle 1

| | Hypervit. A (mg/kg/Tag ) | Papillom Dosis (mg/kg/ Woche) % Aenderung | Therap. Index |
|---|---|---|---|
| | 1. Serie | | |
| 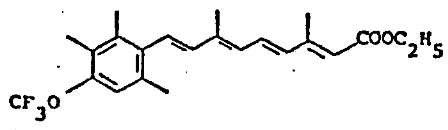 | >400 | 400 - 56<br>200 - 53<br>100 - 29 | $\frac{>400}{200} = 2$ |
| | 2. Serie | | |
| | 400 | 400 - 57<br>200 - 42<br>100 - 27<br>50 - 8 | $\frac{400}{200} = 2$ |
| 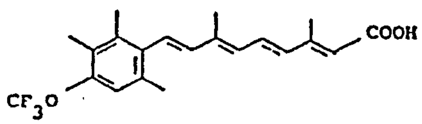 | 50 | 400 - 84<br>200 - 63<br>100 - 16<br>50 - 21<br>25 + 7 | $\frac{50}{150} = 0.3$ |
| 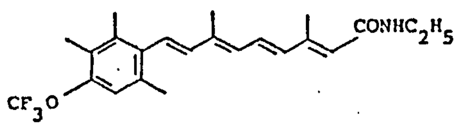 | >400 | 400 - 41<br>200 - 24<br>100 - 18 | $\frac{>400}{>400} = \sim 1$ |
| 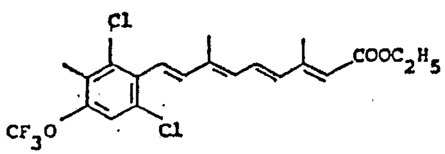 | 200 | 100 - 57<br>50 - 50<br>25 - 12 | $\frac{200}{50} = 4$ |

Die Verbindung der Formel I können als Medikamente in Form pharmazeutischer Präparate, zusammen mit einem verträglichen Trägermaterial Verwendung finden. Beispielsweise können pharmazeutische Präparate für systemische Verabreichung dadurch hergestellt werden, dass man eine Polyenverbindung der Formel I als wirksamen Bestandteil mit pharmazeutisch anwendbaren, nicht toxischen inerten festen oder flüssigen Trägern die allgemein in solchen Präparaten üblich sind, mischt. Die pharmazeutischen Präparate können enteral, parenteral oder topisch verabreicht werden. Geeignete Präparate für die enterale Verabreichung sind beispielsweise Tabletten, Kapseln, Dragées, Sirupe, Suspensionen, Lösungen und Suppositorien. Geeignete Präparate für die parenterale Verabreichung sind Infusionslösungen.

Die Dosierung in der die Verbindungen verabreicht werden können, hängen von der Verabreichungsart und den Bedürfnissen des Patienten ab. Beispielsweise können die Verbindungen in Mengen von 0,5 mg bis 300 mg täglich in einer oder mehreren Dosierungen verabreicht werden.

Die pharmazeutischen Präparate können neben den erfindungsgemässen Wirkstoffen pharmazeutisch anwendbare inerte oder pharmakodynamisch aktive Zusätze enthalten. Beispielsweise können Tabletten oder Granulate eine Reihe von pharmazeutisch anwendbaren Bindemitteln, Füllstoffen, Trägermaterialien oder Verdünnungsmittel enthalten. Flüssige Präparate können beispielsweise als sterile wassermischbare Lösungen vorliegen. Kapseln können einen pharmazeutisch anwendbaren Füllstoff oder Verdicker enthalten. Weiterhin können pharmazeutisch anwendbare geschmacksverbessernde Zusätze und pharmazeutisch anwendbare Substanzen wie Konservierungsmittel, Stabilisatoren, Feuchthaltemittel, Emulgatoren, Salze zur Veränderung des osmotischen Druckes, Puffer, verwendet werden.

Beispiele von pharmazeutisch anwendbaren Trägerstoffen und Verdünnungsmitteln sind Wasser, Gelatine, Lactose, Magnesiumstearat, Talk, Gummi arabicum und Polyalkylenglykole. Für die topische Verabreichung können die erfindungsgemässen Verbindungen als Salben, Tinkturen, Crèmes, Lösungen, Lotionen, Sprays und Suspensionen angewandt werden. Salben, Crèmes und Lösungen sind bevorzugt. Die Präparate können übliche pharmazeutisch anwendbare Antioxidantien, z. B. Tocopherol, n-Methyl-α-tocopheramin, butyliertes Hydroxyaminsol und butyliertes Hydroxytoluol enthalten.

Die folgenden Beispiele erläutern die Erfindung weiter. In den Beispielen ist unter Aether Diäthyläther zu verstehen und die Temperaturen sind in Celsius-Graden angegeben. THF ist Tetrahydrofuran. Raumtemperatur ist etwa 23 °C. Uebliche Aufarbeitung bezeichnet folgendes Verfahren : Das Reaktionsgemisch wurde zwischen Wasser und einem organischen Lösungsmittel, z. B. Diäthyläther oder Methylenchlorid verteilt, die organischen Extrakte wurden mit Wasser oder Kochsalzlösung gewaschen, über Magnesiumsulfat oder Natriumsulfat getrocknet, filtriert und unter vermindertem Druck eingedampft.

## Beispiel 1

201 g 2,3,5-Trimethylphenol wurden in einem Liter Wasser, das 59,2 g Natriumhydroxid enthielt, unter Erwärmen gelöst. Die Lösung wurde filtriert. Eine Lösung von 170 g Thiophosgen (85 % in $CCl_4$) in 900 ml Chloroform wurde auf — 10° gekühlt und unter kräftigem Rühren im Verlauf von 2 Stunden mit der Phenolatlösung so versetzt, dass die Innentemperatur unter 0° blieb. Danach wurde das Reaktionsgemisch weitere 15 Minuten gerührt. Die dunkle Lösung wurde in 1,5 l Hexan und 1 l gesättigte Kochsalzlösung gegossen, die auf 5° vorgekühlt waren. Die organische Phase wurde abgetrennt, über Magnesiumsulfat getrocknet, filtriert und unter vermindertem Druck zu einem rohen öligen Produkt eingedampft, das bei 90-95°/0,3 mm destillierte und 287 g Thiochlorkohlensäure-2,3,5-trimethylphenylester lieferte.

## Beispiel 2

100 g Thiochlorkohlensäure-2,3,5-trimethylphenylester in 1 000 ml Methylenchlorid wurden bei — 35° mit einer Lösung von 20,0 ml Molybdänhexafluorid (Ventron) in 40 ml Methylenchlorid so versetzt, dass die Temperatur nicht über 25° stieg. Danach liess man das schwarze Gemisch auf Zimmertemperatur aufwärmen. Die Hauptmenge des Methylenchlorids wurde abdestilliert, wobei ein schwarzer Rückstand zurückblieb. Destillation des Rückstandes bei 100-180° (Oelbad) lieferte ein Produkt, das in 100 ml Hexan aufgenommen wurde. Die Hexanlösung wurde mit 50 ml 5 % Natriumbicarbonatlösung gewaschen, über Magnesiumsulfat getrocknet, filtriert, eingedampft und destilliert. Man erhielt 22 g 2,3,5-Trimethyl-1-trifluormethoxybenzol, Siedepunkt 76-77°.

## Beispiel 3

Eine Lösung von 31,5 ml 2,3,5-Trimethyl-1-trifluormethoxybenzol, 315 ml Essigsäure, 31,5 ml konzentrierte Salzsäure, 31,5 ml wässriges Formaldehyd und 13 g Chlormethylmethyläther wurde 72 Stunden auf 85° erwärmt. Nach 48 Stunden wurden periodisch Proben aufgearbeitet um das Verhältnis Monochlormethylprodukt : Dichlormethylprodukt : Ausgangsmaterial zu optimieren. Die Lösung wurde dann gekühlt, in 1,5 l Wasser gegossen und mit 3 × 500 ml Hexan extrahiert. Die

Hexanextrakte wurden mit 1 l Wasser und 500 ml gesättigter Natriumbicarbonatlösung gewaschen, über Magnesiumsulfat getrocknet, filtriert und konzentriert. Man erhielt 30 g rohes (4-Trifluormethoxy-2,3,6-trimethylphenyl) methylchlorid, das etwas Ausgangsmaterial enthielt, das durch Destillation bei 24-30°/6.66 Pa entfernt wurde. Der Rückstand, 22 g rohes (4-Trifluormethoxy-2,3,6-trimethylphenyl) methylchlorid wurde direkt im nächsten Reaktionsschritt eingesetzt.

Das rohe (4-Trifluormethoxy-2,3,6-trimethylphenyl) methylchlorid, 27,5 g Triphenylphosphin und 250 ml Acetonitril wurden 20 Stunden zum Rückfluss erhitzt. Das Lösungsmittel wurde bei Raumtemperatur abgedampft und der Rückstand mit 200 ml Aethylacetat verrieben. Der erhaltene Feststoff wurde abfiltriert, in 250 ml heissem Acetonitril gelöst und mit 1,1 l Aethylacetat versetzt. Das Gemisch wurde 2 Stunden stehen gelassen, wobei 38 g (4-Trifluormethoxy-2,3,6-trimethylphenyl) methyltriphenylphosphoniumchlorid ausfielen, Schmelzpunkt 272-274°. Die Mutterlaugen lieferten weitere 10,5 g Produkt.

Beispiel 4

Eine Suspension von 12,2 g (4-Trifluormethoxy-2,3,6-trimethylphenyl) methyltriphenylphosphoniumchlorid in 490 ml trockenem THF wurde auf 45° gekühlt und rasch (binnen 5 Minuten) mit 11;1 ml n-Butyllithium bei —35 bis —45° versetzt. Das Gemisch wurde 15 Minuten bei dieser Temperatur gehalten, langsam auf —25° aufwärmen gelassen, wobei sich die Lösung leicht trüb orange färbte und dann auf —45 bis —50° gekühlt und rasch mit 7 g Aethyl-(2E,4E,6E)-3,7-dimethyl-8-oxo-octatrienoat in 50 ml trockenem THF versetzt. Die Orangefärbung verschwand und das Reaktionsgemisch wurde 2 Stunden bei —25° gerührt, dann im Verlauf von einer Stunde auf 0° aufwärmen gelassen und 2 Stunden bei dieser Temperatur stehen gelassen. Das Gemisch wurde dann in 1 l kaltes Wasser gegossen und mit Hexan extrahiert. Die organischze Phase wurde nacheinander mit Kaltem Wasser, 60 % Methanol-Wasser und gesättigter Kochsalzlösung gewaschen, über Magnesiumsulfat getrocknet, filtriert und konzentriert. Das erhaltene gelbe Oel wurde durch Hochdruckflüssigchromatographie (2 Silicagelsäulen, 2 % Aethylacetat in Hexan als Elutionsmittel) gereinigt und man erhielt 5,79 g (2E,4E,6E,8E)-3,7-dimethyl-9-[2,3,6-trimethyl-4-(trifluormethoxy) phenyl]-nonatetraensäure-äthylester, Schmelzpunkt 47-49°.

Die Hochdruckflüssigchromatographie lieferte weiterhin 1,25 g des 8(Z)-Isomers, (2E,4E,6E,8Z)-3,7-dimethyl-9-[2,3,6-trimethyl-4-(trifluormethoxy) phenyl]-nonatetraensäure-äthylester.

Dieses 8Z-Isomer wurde durch Behandlung mit Jod in Chloroform in das (2E,4E,6E,8E)-Isomer und das 2(Z)-4E,6E,8E-Isomer umgewandelt. Trennung dieses Gemisches durch Hochdruckflüssigchromatographie lieferte weiteres (2E,4E,6E,8E)-Isomer und Fraktionen die mit (2Z,4E,6E,8E)-Isomer angereichert waren. Weitere HPLC-Reinigung der letztgenannten Fraktionen lieferte reinen (2Z,4E,6E,8E)-3,7-Dimethyl-9-[2,3,6-trimethyl-4-(trifluormethoxy) phenyl]-nonatetraensäure-äthylester, der nach Umkristallisation aus Pentan einen Schmelzpunkt von 59-67° zeigte.

Beispiel 5

Ein Gemisch von 15,3 g (2E,4E,6E,8E)-3,7-dimethyl-9-[2,3,6-trimethyl-4-(trifluormethoxy) phenyl]-nonatetraensäure-äthylester, 76,5 ml Dioxan und 30 ml 2N-methanolische KOH wurde 4 Stunden zum Rückfluss erhitzt. Das Gemisch wurde dann in 300 ml Eiswasser gegossen und mit 2N-Schwefelsäure auf pH 2 angesäuert. Der gelbe Niederschdflag wurde mit 3 × 200 ml Chloroform extrahiert, der Extrakt mit Natriumchloridlösung gewaschen und über Magnesiumsulfat getrocknet. Abdampfen des Lösungsmittels lieferte einen gelben Feststoff, der mit 150 ml Hexan behandelt wurde und nach Filtration 12 g rohe (2E,4E,6E,8E)-3,7-Dimethyl-9-[2,3,6-trimethyl-4-(trifluormethoxy) phenyl]-nonatetraensäure lieferte. Umkristallisation aus 200 ml Aethylacetat lieferte 9,25 g (2E,4E,6E,8E)-3,7-Dimethyl-9-[2,3,6-trimethyl-4-(trifluormethoxy) phenyl]-nonatetraensäure vom Schmelzpunkt von 190-193°.

Beispiel 6

Eine Lösung von 2,0 g (2E,4E,6E,8E)-3,7-Dimethyl-9-[2,3,6-trimethyl-4-(trifluormethoxy) phenyl]-nonatetraensäure in 60 ml trockenem THF und 1,6 g Carbonyldiimidazol wurden eine halbe Stunde auf 35-40° erwärmt. Das Reaktionsgemisch wurde auf —10° gekühlt und überschüssiges, gasförmiges Aethylamin eingeleitet. Das erhaltene Gemfisch wurde 1 Stunde bei 23° gerührt, mit 100 ml Hexan verdünnt und über 200 g Silicagel mit Aethylacetat/Hexan (1 : 1) als Elutionsmittel filtriert. Weitere chromatographische Reinigung (Silicagel, 5-10 % Aethylacetat in Methylenchlorid) lieferte einen gelben Feststofff der nach Umkristallisation aus Aethylacetat-Hexan 1,4 g N-Aethyl-(2E,4E,6E,8E)-3,7-dimethyl-9-[2,3,6-trimethyl-4-(trifluormethoxy) phenyl]-nonatetraenamid vom Schmelzpunkt 198-200° lieferte.

Beispiel 7

Eine Lösung von 65 g 3,5-Dichlor-o-cresol in 500 ml 0,75 M-Natronlauge wurde auf 5° gekühlt und unter Rühren zu einer Lösung von 85 g Thiophosgen (85 Vol.-% in $CCl_4$) in 400 ml Chloroform bei 0° im Verldauf einer halben Stunde gegeben. Das Gemisch wurde eine weitere halbe Stunde gerührt und dann

in Eiswasser gegossen. Die organische Phase wurde abgetrennt, mit 500 ml kalter 5 %-iger Natronlauge und 500 ml kalter Kochsalzlösung gewaschen, getrocknet und konzentriert. Der Rückstand wurde destilliert. Man erhielt 73 g Thiochlorkohlensäure-O-2,5-dichlor-3-methylphenylester vom Siedepunkt 107-111°/0,3 mm.

Beispiel 8

Zu einer Lösung von 35 g Thiochlorkohlensäure-O-2,5-dichlor-3-methylphenylester in 250 ml Methylenchlorid wurde bei —35° ein Gemisch von 4,1 ml Molybdänhexafluorid in 20 ml Methylenchlorid gegeben. Das Reaktionsgemisch wurde im Verlaufe einer Stunde auf 20° aufwärmen gelassen und wie in Beispiel 2 bei der Herstellung von 2,3,5-Trimethyl-1-trifluormethoxybenzol beschrieben, aufgearbeitet. Wiederholte Destillation lieferte 10,3 g 1,3-Dichlor-5-(trifluormethoxy)-4-methylbenzol vom Schmelzpunkt 69-71°.

Beispiel 9

8,0 g 1,3-Dichlor-5-(trifluormethoxy)-4-methylbenzol und 5,0 g Chlormethylmethyläther wurden unter Rühren zu 50 ml 98 %-iger Schwefelsäure bei 20° gegeben. Das Gemisch wurde 15 Stunden gerührt, dann in 200 ml Eiswasser gegossen und mit 2 × 100 ml Hexan extrahiert. Die Hexanextrakte wurden mit 100 ml Wasser gewaschen, getrocknet, filtriert und konzentriert. Der Rückstand wurde in 50 ml Hexan gelöst, filtriert und konzentriert. Der erhaltene weisse Feststoff wurde bei 25° unter 0,001 mm getrocknet und lieferte 9,0 g 1,3-Dichlor-2-chlormethyl-4-methyl-5-(trifluormethoxy)-benzol.

Beispiel 10

Eine Lösung von 8,0 g 1,3-Dichlor-2-chlormethyl-4-methyl-5-(trifluormethoxy) benzol in 250 ml Toluol wurde mit 15,0 g Triphenylphosphin versetzt und 90 Stunden auf 90-95° erwärmt. Das während des Erwärmens gebildete feste Produkt wurde von Zeit zu Zeit abfiltriert. Die Feststoffe wurden vereinigt und getrocknet (60°/0,001 mm) und lieferten 12,0 g [2,6-Dichlor-3-methyl-4-(trifluormethoxy phenyl] methyl]-triphenylphosphoniumchlorid.

Beispiel 11

Eine Lösung von 5,1 g des im Beispiel 10 hergestellten Phosphoniumsalzes in 150 ml trockenem THF wurde auf —70° gekühlt und mit 4,8 ml n-Butyllithium (2,3 M in Hexan) versetzt. Das Gemisch wurde 15 Minuten bei —70° gerührt und dann mit einer Lösung von 4,0 g (2E,4E,6E,8E)-3,7-Dimethyl-8-oxo-octatriensäureäthylester in 15 ml THF versetzt. Das Gemisch wurde langsam auf 0° aufwärmen gelassen, und 4 Stunden bei dieser Temperatur gehalten, dann in 200 ml Wasser gegossen und mit 2 × 200 ml Hexan extrahiert. Die Hexanextrakte wurden vereinigt und mit 2 × 150 ml 60 %-igem wässrigem Methanol und 150 ml gesättigter Kochsalzlösung gewaschen. Die Hexanschicht wurde getrocknet, filtriert und eingedampft. Reinigung durch HPLC (2 Silicagelsäulen, 5 % Aethylacetat in Hexan als Elutionsmittel) und Kristallisation aus Hexan lieferten 1,65 g (2E,4E,6E,8E)-3,7-Dimethyl-9-[2,6-dichloro-3-methyl-4-(trifluormethoxy) phenyl]-nonatetraensäureäthylester in Form hellgelber Kristalle vom Schmelzpunkt 93-94°.

Beispiel 12

In Analogie zu Beispiel 5 wurden 0,04 Mol (2E,4E,6E,8E)-3,7-Dimethyl-9-[2,6-dichlor-3-methyl-4-(trifluormethoxy)-phenyl]-nonatetraensäureäthylester in Dioxan mit 2N methanolischer KOH 4 Stunden zum Rückfluss erhitzt. Ansäuern und Aufarbeitung lieferte (2E,4E,6E,8E)-3,7-Dimethyl-9-[2,6-dichlor-3-methyl-4-(trifluormethoxy) phenyl]-nonatetraensäure.

Beispiel 13

In Analogie zu Beispiel 6 wurde eine Lösung von 5 mMol (2E,4E,6E,8E)-3,7-Dimethyl-9-[2,6-dichlor-3-methyl-4-(trifluormethoxy) phenyl]-nonatetraensäure in trockenem THF eine halbe Stunde bei 35-40° mit 10 mMol Carbonyldiimidazol behandelt. Das Reaktionsgemisch wurde auf —10° gekühlt und es wurde gasförmiges Aethylamin im Ueberschuss eingeleitet. Nach 1 Stunde wurde das Reaktionsgemisch aufgearbeitet und man erhielt N-Aethyl-(2E,4E,6E,8E)-3,7-dimethyl-9-[2,6-dichlor-3-methyl-4-(trifluormethoxy) phenyl]-nonatetraenamid.

Beispiel 14

Durch Behandlung einer Lösung von (2E,4E,6E,8E)-3,7-dimethyl-9-[2,3,6-trimethyl-4-(trifluormethoxy) phenyl]-nonatetraensäure-äthylester in Methylenchlorid mit einer Lösung von Diisobutylaluminiumhydrid in Hexan bei —60° unter Argon und Aufarbeitung des Reaktionsgemisches erhält man (2E,4E,6E,8E)-3,7-Dimethyl-9-[2,3,6-trimethyl-4-(trifluormethoxy) phenyl]-nonatetraen-1-ol.

Beispiel 15

Durch Behandlung einer Lösung von 0,6 mMol (2E,4E,6E,8E)-3,7-Dimethyl-9-[2,3,6-trimethyl-4-(trifluormethoxy)-phenyl]-nonatetraen-1-ol in 20 ml Methylchlorid mit einer Suspension von 1,1 g aktiviertem Mangandioxid in 40 ml Methylenchlorid und Aufarbeitung des Reaktionsgemisches erhält man (2E,4E,6E,8E)-3,7-Dimethyl-9-[2,3,6-trimethyl-4-(trifluormethoxy) phenyl]-nonatetraen-1-al.

Beispiel 16

In Analogie zu Beispiel 14 kann (2E,4E,6E,8E)-3,7-Dimethyl-9-[2,6-dichlor-3-methyl-4-(trifluormethoxy)-phenyl]-nonatetraensäure (31 mMol) in 100 ml Methylenchlorid bei —60° mit 69 mMol Isobutylaluminiumhydrid reduziert werden. Aufarbeitung des Reaktionsgemisches liefert (2E,4E,6E,8E)-3,7-Dimethyl-9-[2,6-dichlor-3-methyl-4-(trifluormethoxy) phenyl]-nonatetraen-1-ol.

Beispiel 17

Durch Umsetzung einer Lösung einer Lösung von (2E,4E,6E,8E)-3,7-Dimethyl-9-[2,6-dichlor-3-methyl-4-(trifluormethoxy)-phenyl]-nonatetraen-1-ol in 20 ml Methylenchlorid mit einer Suspension von 1,1 g aktiviertem Mangandioxid in 40 ml Methylenchlorid erhält man nach Aufarbeitung das (2E,4E,6E,8E)-3,7-Dimethyl-9-[2,6-dichlor-3-methyl-4-(trifluormethoxy)-phenyl]-nonatetraen-1-al.

Beispiel 18

Kapselformulierungen mit (2E,4E,6E,8E)-3,7-dimethyl-9-[2,3,6-trimethyl-4-(trifluormethoxy) phenyl]-nonatetreaensäure-äthylester als Wirkstoff

| | mg/Kapsel | | | | | |
|---|---|---|---|---|---|---|
| Wirkstoff | 0,5 | 5,0 | 25,0 | 50,0 | 100,0 | 300,0 |
| BHA | 0,01 | 0,02 | 0,05 | 0,1 | 0,1 | 0,1 |
| BHT | 0,01 | 0,02 | 0,05 | 0,1 | 0,1 | 0,1 |
| Kokosöltriglyzeridfraktionen | 130,0 | 130,0 | 200,0 | 250,0 | 500,0 | 900,0 |
| | — | — | — | — | — | — |
| Kapselgewicht | 130,52 | 135,04 | 225,1 | 300,2 | 600,2 | 1 200,2 |

Die Inhaltsstoffe werden in dem warmen Triglyzerid gründlich gemischt und danach in Weichgelatinekapseln geeigneter Grösse abgefüllt.

**Patentansprüche**

1. Verbindungen der allgemeinen Formel

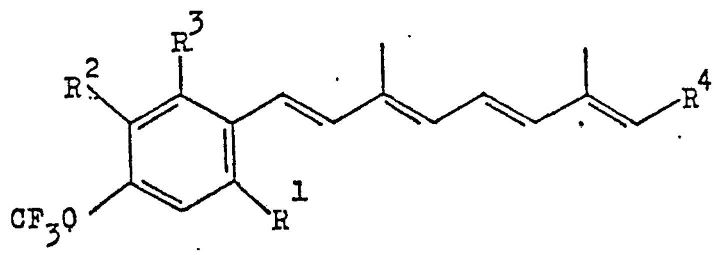

(I)

worin
$R^1$ nieder-Alkyl oder Halogen,
$R^2$ nieder-Alkyl,
$R^3$ nieder-Alkyl oder Halogen,

$R^4$ —$CH_2$—OH, 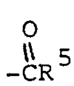 oder 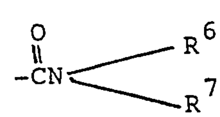

$R^5$ Wasserstoff, Hydroxy oder nieder-Alkoxy und $R^6$ und $R^7$ Wasserstoff oder nieder-Alkyl bedeuten, und Salze von Carbonsäuren der Formel I.

2. Verbindungen gemäss Anspruch 1, in denen die Seitenkette all-trans ist.

3. Verbindungen gemäss den Ansprüchen 1 oder 2, in denen mindestens einer der Reste $R^1$ und $R^3$ nieder-Alkyl oder Halogen ist.

4. Verbindudngen gemäss den Ansprüchen 1-3, in denen $R^4$ nieder-Alkoxycarbonyl oder —C(O)N($R^6$,$R^7$) ist.

5. (2E,4E,6E,8E)-3,7-Dimethyl-9-[2,3,6-trimethyl-4-(trifluormethoxy) phenyl] nonatetraensäure.

6. (2E,4E,6E,8E)-3,7-Dimethyl-9-[2,3,6-trimethyl-4-(trifluormethoxy) phenyl] nonatetraensäure-äthylester.

7. (2E,4E,8E)-3,7-Dimethyl-9-[2,6-dichloro-3-methyl-4-(trifluormethoxy) phenyl] nonatetraensäure-äthylester.

8. N-Aethyl-(2E,4E,6E,8E)-3,7-dimethyl-9-[2,3,6-trimethyl-4-(trifluormethoxy) phenyl] nonatetraenamid.

9. Verbindungen gemäss Anspruch 1 zur Verwendung als Heilmittel.

10. Verbindungen gemäss Anspruch 1 zur Verwendung bei der Behandlung von Tumoren, Akne oder Psoriasis.

11. Pharmazeutisches Präparat, enthaltend eine Verbeidung gemäss Anspruch 1.

12. Verfahren zur Herstellung von Verbindungen der Formel I gemäss Anspruch 1 und deren Salzen, dadurch gekennzeichnet, dass man eine Verbindung der Formel

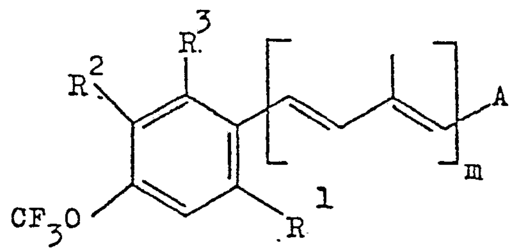

(II)

mit einer Verbindung der Formel

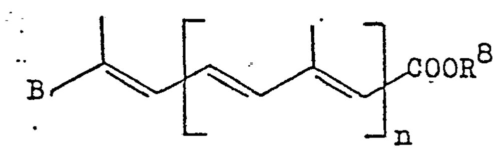

(III)

worin $R^1$, $R^2$ und $R^3$ die in den Ansprüchen 1 und 3 genannte Bedeutung haben, eines der Symbole A und B Formyl ist und das andere eine Gruppe der Formeln

$$-CH_2-\overset{\oplus}{P}(Ar)_3Y^{\ominus} \quad \text{oder} \quad -CH_2-\overset{O}{\overset{\|}{P}}(OR^9)_2$$

darstellt, Ar Aryl ist, $Y^{\ominus}$ das Anion einer organischen oder anorganischen Säure und $R^8$ und $R^9$ nieder-Alkyl sind, wobei m = 0 und n = 1 oder m = 1 und n = 0 sind, umsetzt und gewünschtenfalls eine nieder-Alkoxycarbonylgruppe —COOR$^8$ in einer nerhaltenen Verbindung der Formel I in eine Carboxyl, Formyl, Hydroxymethyl, Carboxamid oder mono- oder Dialkylcarboxamidgruppe umwandelt und gewünschtenfalls eine erhaltene Carbonsäure in ein pharmazeutisch anwendbares Salz umwandelt.

13. Verfahren nach Anspruch 12, dadurch gekennzeichnet, dass in den Verbindungen der Formeln II und III

$$A \; -CH_2\overset{\oplus}{P}(Ar)_2Y^{\ominus} \quad \text{oder} \quad -CH_2\overset{O}{\overset{\uparrow}{P}}(OR^9)_2,$$

B Formyl, m 0 und n 1 ist.

14. Verfahren nach Anspruch 12 oder 13, dadurch gekennzeichnet, dass man die all-trans-Verbindungen der Formel I in reiner Form isoliert.

15. Verfahren nach den Ansprüchen 12-14, dadurch gekennzeichnet, dass mindestens ein Rest $R^1$ oder $R^3$ nieder-Alkyl oder Halogen ist.

16. Verfahren nach den Ansprüchen 12-15, dadurch gekennzeichnet, dass $R^4$ nieder-Alkoxycarbonyl oder —C(O)N($R^6$,$R^7$) ist.

17. Verfahren nach den Ansprüchen 12-16, dadurch gekennzeichnet, dass man (2E,4E,6E,8E)-3,7-Dimethyl-9-[2,3,6-trimethyl-4-(trifluormethoxy) phenyl] nonatetraensäure herstellt.

18. Verfahren nach den Ansprüchen 12-16, dadurch gekennzeichnet, dass man (2E,4E,6E,8E)-3,7-Dimethyl-9-[2,3,6-trimethyl-4-(trifluormethoxy) phenyl] nonatetraensäure-äthylester herstellt.

19.Verfahren nach den Ansprüchen 12-16, dadurch gekennzeichnet, dass man (2E,4E,6E,8E)-3,7-Dimethyl-9-[2,6-dichloro-3-methyl-4-(trifluormethoxy) phenyl] nonatetraensäure-äthylester herstellt.

20. Verfahren nach den Ansprüchen 12-16, dadurch gekennzeichnet, dass man N-Aethyl-(2E,4E,6E,8E)-3,7-dimethyl-9-[2,3,6-trimethyl-4-(trifluormethoxy) phenyl] nonatetraenamid herstellt.

**Claims**

1. A compound of the formula

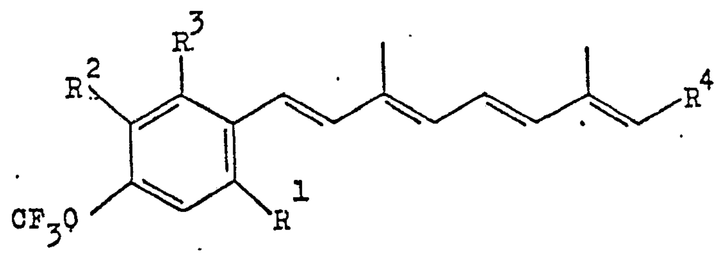

(I)

wherein

$R^1$ is lower alkyl or halo ;

$R^2$ is lower alkyl ;

$R^3$ is lower alkyl or halo ;

$R^4$ is —$CH_2OH$, 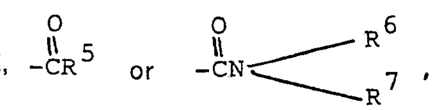
or
,

$R^5$ is hydrogen, hydroxy or lower alkoxy and $R^6$ and R7 each are hydrogen or lower alkyl, and salts of carboxylic acids of the formula I.

2. The compounds of claim 1 wherein the side chain is all trans.

3. The compounds of claim 1 or 2 wherein at least one of $R^1$ and $R^3$ is low alkyl or halo.

4. The compounds of claims 1 to 3, wherein $R^4$ is lower alkoxycarbonyl or —C(O)N($R^6$,$R^7$).

5. (2E,4E,6E,8E)-3,7-Dimethyl-9-[2,3,6-trimethyl-4-(trifluoromethoxy) phenyl] nonatetraenoic acid.

6. Ethyl-(2E,4E,6E,8E)-3,7-dimethyl-9-[2,3,6-trimethyl-4-(trifluoromethoxy) phenyl] nonatetraenoate.

7. (2E,4E,6E,8E)-3,7-Dimethyl-9-[2,6-dichloro-3-methyl-4-(trifluoromethoxy) phenyl] nonatetraenoic acid ethyl ester.

8. N-Ethyl (2E,4E,6E,8E)-3,7-dimethyl-9-[2,3,6-trimethyl-4-(trifluoromethoxy) phenyl] nonatetraenamide.

9. The compounds of claim 1 for use as a pharmaceutical.

10. The compounds of claim 1 for use in the treatment of tumours, acne or psoriasis.

11. A pharmaceutical composition containing a compound as claimed in claim 1.

12. Process for the preparation of compounds of formula I as claimed in claim 1 characterized in that a compound of formula

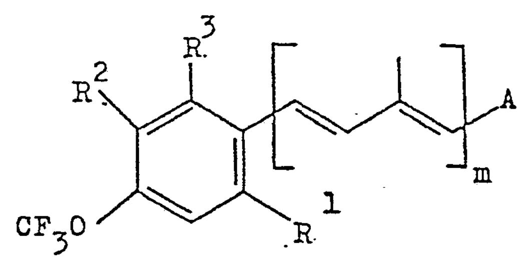

(II)

is reacted with a compound of formula

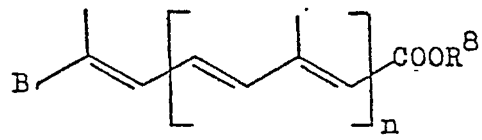

(III)

wherein $R^1$, $R^2$ and $R^3$ are as in claims 1 and 3, one of the symbols A and B is formyl and the other is a group of the formula

$$-CH_2-\overset{\oplus}{P}(Ar)_3Y^{\ominus} \quad \text{or} \quad -CH_2-\overset{O}{\overset{\|}{P}}(OR^9)_2$$

Ar is Aryl ; $Y^{\ominus}$ is an anion of an organic or anorganic acid ; $R^8$ and $R^9$ are lower alkyl, and, if desired, converting the lower alkoxy carbonyl group —$COOR^8$ in the resulting compound of formula I into a carboxyl, formyl, hydroxymethyl, carboxamide or mono- or dialkylcarboxamide group and if desired, converting a carboxylic acid obtained into a pharmaceutically acceptable salt.

13. A process as in claim 12, wherein in the compounds of formula II and III, A is

$$A\ -CH_2\overset{\oplus}{P}(Ar)_2Y^{\ominus} \quad \text{or} \quad -CH_2\overset{O}{\overset{\uparrow}{P}}(OR^9)_2,$$

B is formyl, m is 0 and n is 1.

14. A process as in claim 12 or 13, wherein the all-trans compounds of formula I are isolated in pure form.

15. A process as in any one of claims 12 to 14 wherein at least one of $R^1$ and $R^3$ is lower alkyl or halo.

16. A process as in any one of claims 12 to 15 wherein $R^4$ is lower alkoxycarbonyl or —$C(O)N(R^6,R^7)$.

17. A process as in any one of claims 12 to 16 wherein (2E,4E,6E,8E)-3,7-dimethyl-9-[2,3,6-trimethyl-4-(trifluoromethoxy) phenyl] nonatetraenoic acid is prepared.

18. A process as in any one of claims 12 to 16 wherein ethyl-(2E,4E,6E,8E)-3,7-dimethyl-9-[2,3,6-trimethyl-4-(trifluoromethoxy) phenyl] nonatetraenoate is prepared.

19. A process as in any one of claims 12 to 16 wherein (2E,4E,6E,8E)-3,7-dimethyl-9-[2,6-dichloro-3-methyl-4-(triflfuoromethoxy) phenyl] nonatetraenoic acid ethyl ester is prepared.

20. A process as in any one of claims 12 to 16 wherein N-ethyl (2E,4E,6E,8E)-3,7-dimethyl-9-[2,3,6-trimethyl-4-(trifluoromethoxy) phenyl] nonatetraenamide is prepared.

**Revendications**

1. Composés de formule générale

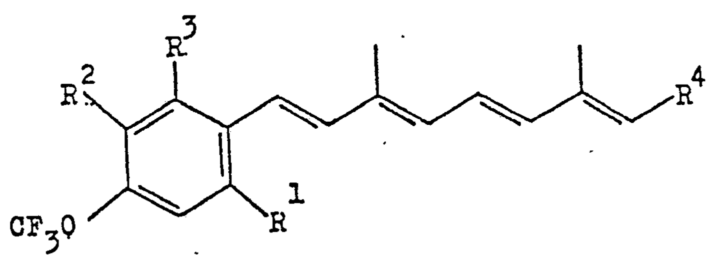

(I)

dans laquelle

$R^1$ représente un groupe alkyle inférieur ou un halogène,

$R^2$ représente un groupe alkyle inférieur,

$R^3$ représente un groupe alkyle inférieur ou un halogène,

$R^4$ représente

$$—CH_2—OH,\ -\overset{O}{\overset{\|}{C}}R^5 \ \text{ou}\ -\overset{O}{\overset{\|}{C}}N\begin{matrix}R^6\\R^7\end{matrix},$$

$R^5$ représente l'hydrogène, un groupe hydroxy ou alcoxy inférieur et $R^6$ et $R^7$ représentent l'hydrogène ou des groupes alkyle inférieurs,

et les sels des acides carboxyliques de formule I.

2. Composés selon la revendication 1, dans lesquels la chaîne latérale est entièrement trans.

3. Composés selon les revendications 1 ou 2, dans lesquels au moins un des symboles $R^1$ et $R^3$ représente un groupe alkyle inférieur ou un halogène.

4. Composés selon les revendications 1 à 3, dans lesquels $R^4$ représente un groupe (alcoxy inférieur)-carbonyle ou $—C(O)N(R^6,R^7)$.

5. L'acide (2E,4E,6E,8E)-3,7-diméthyl-9-[2,3,6-triméthyl-4-(trifluorométhoxy)-phényl]-nonatétraénoïque.

6. Le (2E,4E,6E,8E)-3,7-diméthyl-9-[2,3,6-triméthyl-4-(trifluorométhoxy)-phényl]-nonatétraénoate d'éthyle.

7. Le (2E,4E,8E)-3,7-diméthyl-9-[2,6-dichloro-3-méthyl-4-(trifluorométhoxy)-phényl]-nonatétraénoate d'éthyle.

8. Le N-éthyl-(2E,4E,6E,8E)-3,7-diméthyl-9-[2,3,6-triméthyl-4-(trifluorofméthoxy)-phényl]-nonatétraénamide.

9. Composés selon la revendication 1, pour l'utilisation en tant que médicaments.

10. Composés selon la revendication 1, pour l'utilisation dans le traitement des tumeurs, de l'acné ou du psoriasis.

11. Composition pharmaceutique contenant un composé selon la revendication 1.

12. Procédé de préparation des composés de formule I selon la revendication 1 et de leurs sels, caractérisé en ce que l'on fait réagir un composé de formule

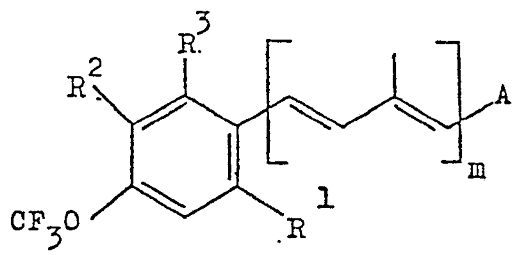

(II)

avec un composé de formule

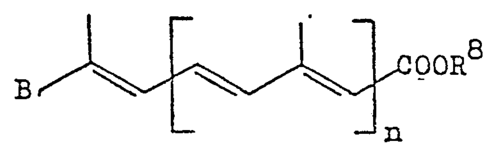

(III)

les symboles $R^1$, $R^2$ et $R^3$ ayant les significations indiquées dans les revendications 1 et 3, l'un des symboles A et B représentant un groupe formyle et l'autre un groupe de formule

$$-CH_2-\overset{\oplus}{P}(Ar)_3Y^{\ominus} \quad \text{ou} \quad -CH_2-\overset{O}{\overset{\|}{P}}(OR^9)_2$$

Ar représente un groupe aryle, $Y^{\ominus}$ est l'anion d'un acide organique ou minéral et $R^8$ et $R^9$ représentent des groupes alkyle inférieurs, $m = 0$ et $n = 1$ ou $m = 1$ et $n = 0$.
et si on le désire, on convertit un groupe (alcoxy inférieur)-carbonyle $—COOR^8$ contenu dans un composé de formule I ainsi obtenu en un groupe carboxyle, formyle, hydroxyméthyle, carboxamide ou mono- ou dialkylcarboxamide, et si on le désire, on convertit un acide carboxylique ainsi obtenu en un sel acceptable pour l'usage pharmaceutique.

13. Procédé selon la revendication 12, caractérisé en ce que, dans les composés de formules II et III, A représente

$$A\ -CH_2\overset{\oplus}{P}(Ar)_2Y^{\ominus} \quad \text{ou} \quad -CH_2\overset{O}{\overset{\uparrow}{P}}(OR^9)_2,$$

B représente un groupe formyle, m est égal à 0 et n est égal à 1.

14. Procédé selon la revendication 12 ou 13, caractérisé en ce que l'on isole les composés entièrement trans de formule I à l'état pur.

15. Procédé selon les revendications 12 à 14, caractérisé en ce que l'un au moins des symboles $R^1$ ou $R^3$ représente un groupe alkyle inférieur ou un halogène.

16. Procédé selon les revendications 12 à 15, caractérisé en ce que $R^4$ représente un groupe (alcoxy inférieur)-carbonyle ou —C(O)N($R^6$,$R^7$).

17. Procédé selon les revendications 12 à 16, caractérisé en ce que l'on prépare l'acide (2E,4E,6E,8E)-3,7-diméthyl-9-[2,3,6-triméthyl-4-(trifluorométhoxy)-phényl]-nonatétraénoïque.

18. Procédé selon les revendications 12 à 16, caractérisé en ce que l'on prépare le (2E,4E,6E,8E)-3,7-diméthyl-9-[2,3,6-triméthyl-4-(trifluorométhoxy)-phényl]-nonatétraénoate d'éthyle.

19. Procédé selon les revendications 12 à 16, caractérisé en ce que l'on prépare le (2E,4E,6E,8E)-3,7-diméthyl-9-[2,6-dichloro-3-méthyl-4-(trifluorométhoxy)-phényl]-nonatétraénoate d'éthyle.

20. Procédé selon les revendications 12 à 16, caractérisé en ce que l'on prépare le N-éthyl-(2E,4E,6E,8E)-3,7-diméthyl-9-[2,3,6-triméthyl-4-(trifluorométhoxy)-phényl]-nonatétraénamide.